# EUROPEAN PATENT SPECIFICATION

(11) **EP 1 688 122 B1**
(45) Date of publication and mention of the grant of the patent: **20.08.2008**
(21) Application number: 05002445.4
(22) Date of filing: 04.02.2005
(51) Int. Cl.: A61H 37/00, A61H 33/04

(54) **Sensory experience method and apparatus**
Vorrichtung und Verfahren zur sensorischen Wahrnehmung
Dispositif et méthode pour perception sensorielle

(43) Date of publication of application: 09.08.2006
(73) Proprietor: Klein, Jurgen, Kailua Hawaii 96734 (US)
(72) Inventor: Klein, Jurgen, Kailua Hawaii 96734 (US)
(74) Representative: Wolff, Felix

(56) References cited:
- EP-A- 0 098 390
- EP-A- 0 986 985
- WO-A-02/36063
- US-A- 4 114 205
- US-A- 4 424 598
- US-A- 4 640 266

## Description

This invention relates to both an apparatus in the one case and a method in the other for effecting a beneficial sensory experience for a human participant.

The problem to which this invention is directed relates to both a method and apparatus for providing an olfactory and visual stimulation while reducing other stimulation so as to heighten the positive stimulatory effects.

It is known to provide relaxation by having a participant float within a body of high-density liquid such as a highly salted water bath.

While floating, the body is supported more or less uniformly so that the effect of gravity is distributed more uniformly so that individual muscle tensions to say, support a limb, can be reduced and especially if the liquid is at about body temperature, then stimuli to the floating body requiring compensation causing possible further tensions for heating or cooling of the body is reduced allowing the brain to focus on other inputs.

It is however, difficult to know what further stimulation might be most appropriate for any individual for instance in the form of visual or olfactory stimulation.

Document EP-A1-986 985 describes a bathingtub or a shower with means for adding aromatic substances to the water, generating coloured lights and diffusing music sounds.

My invention, as it results from the claims 1 and 12, is that it is feasible to provide over a flotation bath an environment which includes an atmosphere of fine water droplets which can be used for intersecting and reflecting light, but also act as a carrier both to introduce and then assist in extraction of an essential oil.

The advantage of this is that, whereas essential oils once introduced into an enclosure would be expected to persist, if they are carried within an atmosphere of fine mist which is fine water droplets then the droplets themselves are more able to be directed and will then be appropriate to both carry in, hold in a dispersed state and then be reasonably effectively extracted by being carried with the water droplets.

What this means then is that there can be an exchange over a relatively smaller period of time of an essential oil.

There can be also a relationship between other sensory inputs and essential oils.

Such other in puts can include a colour or colour combination and in another case it can either be an audible sound or combination of sounds or of course such sounds in the form of music.

Accordingly, according to one form of this invention, there is then proposed a method of stimulation of the senses for a human participant, the method comprising the steps of having the human participant being supported either directly or indirectly by a body of liquid, and, while floating, having at least stimulation provided by an essential oil or oils combination, and as well, a visual stimulation at the same time, which is provided by a source or sources of light providing light which is directed into an atmosphere of fine water droplets, where the essential oil or oils are carried and dispersed within the fine water droplets.

In preference, the method includes the further step while the human participant remains floating within the body of liquid, that a first essential oil or oil combination is introduced with or during introduction into an enclosure within which the participant exists and at a selected time thereafter, there is extracted from the enclosure at least to a substantive extent the fine water droplets together with such essential oils that have been collected by such fine water droplets, and thereafter at a further selected time, introducing a further atmosphere of fine water droplets and a further essential oil or oils combination during or with at least this further fine droplet atmosphere.

In preference, during at least some of the period during which a first of the fine droplets have been introduced and are left to remain within the enclosure, there is also provided an input of an audible sound.

In preference, the audible sound is in the form of music which has been selected to be appropriate to be with the essential oil or oils combination at the time.

In preference, there is a source of light which is coloured so that the coloured light is directed to shine within the enclosure at the least while the fine water droplets are suspended in the atmosphere within the enclosure, and the colour or colours of such light are selected to be appropriate to the essential oil or oils combination within the enclosure at the time.

In preference, the pattern of introducing and extracting an atmosphere of fine water droplets and an accompanying essential oil or oils combination is repeated a plurality of times with a period between such different sets of atmosphere to allow for effective extraction and then further introduction of fresh fine water droplets.

In a further form, the invention can be said to reside in an apparatus for assisting in providing beneficial stimulation comprising a body of water within an enclosure, means to introduce into the enclosure a first injection of fine water droplets, a means to introduce into the enclosure at least at sometime during the injection of the said first fine water droplets an essential oil or oils combination, and means to extract from the enclosure to at least a substantive extent fine water droplets within the atmosphere of the enclosure, a means then to effect a further introduction of fine water droplets into the atmosphere of the enclosure with a second essential oil or oils combination, after a selected period of time from the said first extraction of fine water droplets from the enclosure.

In preference, there is provided a sequenced arrangement for introduction and extraction of fine water droplets together with a sequence of introduction of a selected essential oil or oils combination with each further introduction of fine water droplets.

In preference, there are means to introduce the essential oil or oils into the stream of fine water particles as they are introduced into the enclosure so that at least during some of the time of such introduction, the essential oil or oils combination will be intimately mixed with such fine water particles or droplets.

In preference, there are means to effect a source of light which will effect a lighting of the atmosphere within the enclosure and there are means to effect a colouring of such light from its source which colour is selected for each of the introduced essential oils.

In other words, there will be a matching sequence of essential oil, colours of lighting and as well a matching audio input.

In preference, there are means to effect a heating of the liquid within the body of water and means to maintain this at a selected temperature which can be at about body temperature.

In preference, the body of liquid is a high salt content and in preference such salt is Epsom salts which is to an extent that a human body will float with a substantive proportion of the human body above a level of the liquid in the bath.

In preference, a person would use a flotation pillow for their head if desired and also would have closures for the ears such that liquid would not pass into the ear but such closures would be selected to allow for hearing of audible signals and music through these closures.

In preference, there would be a sequence control which would allow for a selected time for introduction of water vapour with appropriate essential oil or oils combination, a period during which the fine water droplets would be allowed then to remain dormant while the colours are shone through the atmosphere of the enclosure and the sound is played, and then there is a period for extraction and reinsertion of a fresh set of fine water droplets together with a different set of essential oil or oils combination and at the same time a change in the audible sounds and also the colours and patterns selected for this particular further essential oil.

In preference there can be a sequence of these over a period which may be typically a one-hour stretch and six full changes through such a period so that each cycle is through a period of ten minutes.

For a better understanding of this invention it will now be described with reference to a preferred embodiment which shall be described with the assistance of drawings wherein,
Fig 1 is a schematic view showing an enclosure with a pool, a person shown typically within the pool, and associated accessories relating to all of these according to the embodiment,
Fig 2 illustrates a program pattern of introduction of the stimuli according to this embodiment and according to a selected program.

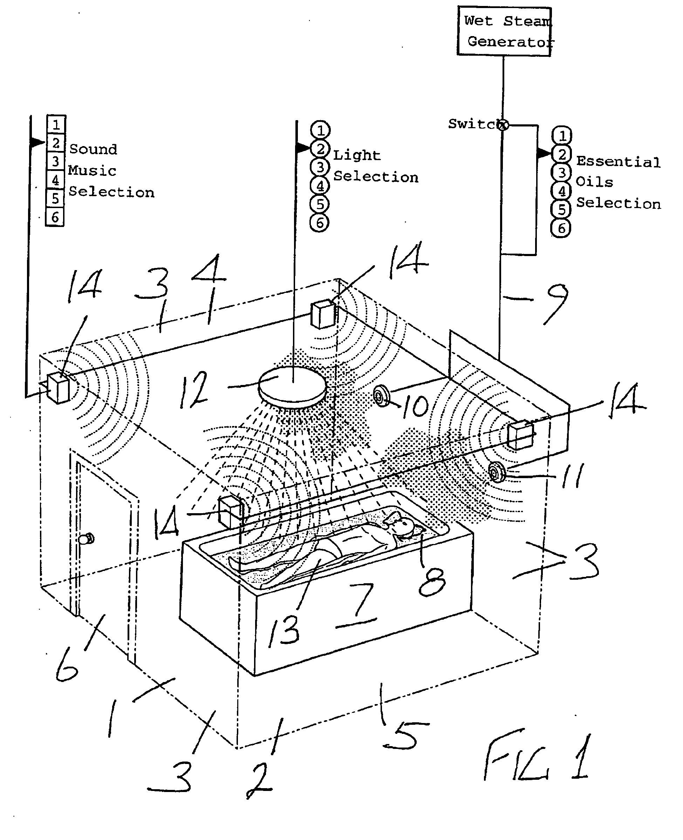

Now referring to the drawings and in particular to Fig 1, there is an arrangement 1 which includes an enclosure 2 which is defined by four walls 3 and a ceiling 4 and of course a floor 5 with access thereto by a door 6.

Within the enclosure thus defined there is a pool 7 which includes liquid 8 which in this case is water with a high concentration of Epsom salts to the extent that a participant can float readily within the liquid without assistance and such that a substantive portion of the body is above the level of the liquid.

In our embodiment, additional buoyancy support can be provided for the head of the participant and it has advantage to provide closures against liquid egress into the ear canals, but selected so that sound can still pass generally through such closures. Such devices are commonly available.

There are means to both introduce and extract fine water droplets this being a - conduit where the conduit in this case is being shown at 9 and the inlets and outlets being shown at 10 and 11.

There are various ways that fine water droplets can be generated and one of these is to create steam and to allow some of this to condense which then provides a fine mist or fine water droplets which generally then will suspend in conventional atmosphere.

There is also the additional advantage that the fine water droplets maintain a temperature that is at least warm and this then provides an environment that is generally at about a body temperature.

By having fine water droplets in the atmosphere of the enclosure 2, this has two functions, one of which is to carry and distribute essential oils and then secondly, to facilitate the extraction of essential oils after a period of time so that a replacement essential oil without at least substantive contamination of the first essential oil can be achieved.

While reference is made to one essential oil it is possible to use a combination of essential oils at the same time.

However, I have found that there is a beneficial combination of various essential oils and colours of light and in this embodiment then there is provided that there are means to effect a combination of an essential oil selection and a colour of light selection so that these are matched for any particular selected period.

The provision of light is shown in our illustration by a light source 12 and there are means specifically within the light source 12 that control and effect a change in sequence with the selection of essential oils such that there is a matching light for each of the essential oils chosen.

Such devices can be obtained from conventional commercial sources and are typically used in theatrical productions and night club environments.

In conjunction with the combination of essential oils and light colour, there is also an additional input namely, music which is also selected to match the colour and essential oil in a particular phase of the stimulation period.

There can be either music chosen or a tone or tone combination in this embodiment and these are able to be selected prior to the participant being involved in the stimulatory program.

However, in a further embodiment, there are means for the participant to effect a further selection and to interact or change as desired either the length of the stimulation, or a particular combination.

In this particular embodiment there can be means accessible by the participant 13.

There are a plurality of loud speakers at 14 which project sound into the enclosure 2 either in multi-channel form or in mono oral form.

One of the difficulties with the arrangement described is that an essential oil when introduced into an enclosure will normally distribute over many of the surfaces and remain generally within the atmosphere of the enclosure over an extended period of time.

By using a wet steam which produces fine droplets and In this particular case introducing this so that an injected liquid namely a selected essential oil or oils into the wet steam, at least through only a portion of the period of introduction of the steam into the enclosure, a substantive portion of the essential oil will remain with the liquid and by extracting the wet steam from the enclosure 2, there can then be expected to be at least a substantive, if not total, removal of the phase selected essential oil.

Not shown but included within the enclosure is also a fresh air inlet which can also include a means for heating such fresh air to maintain a body temperature of the environment within the enclosure and this can assist then in firstly providing a through flow for steam removal together with the accompanying essential oil.

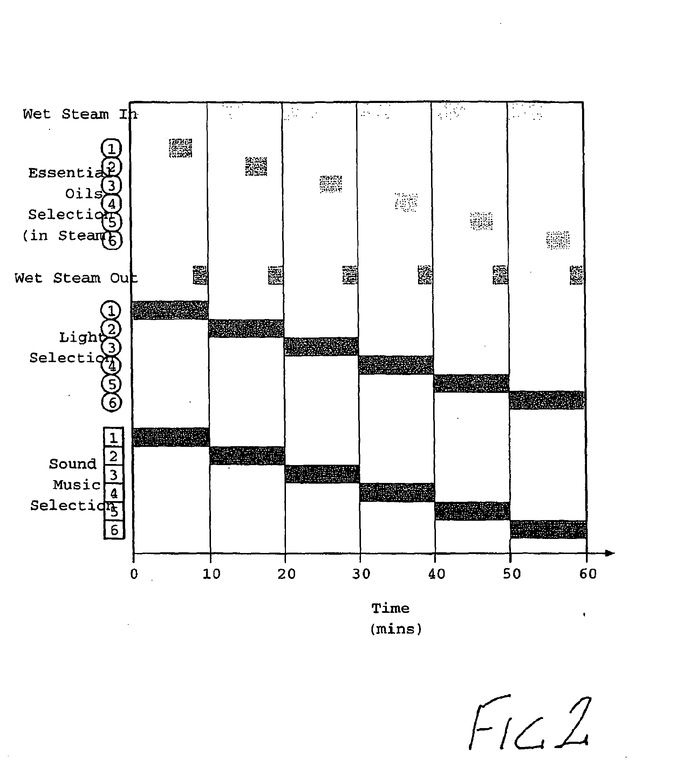

Fig 2 illustrates in graphical form a program according to the embodiment in which there is provided over a 60 minute period, a sequence of in any ten minute period, a first supply of wet steam in, and during such supply of set steam in, a smaller period during which there is opened a valve into a supply of selected essential oil and by use of suction, a selected proportion of essential oil is thereby pulled into and mixed up with and dissolved by the liquid within the wet steam.

At the same time through the selected phase, there is a coloured light which is arbitrarily shown as selection 1, and there is a sound choice also as selection 1.

Each of the subsequent phases match the same pattern where there is a selection of essential oils, a colour or as preferred a light pattern with colour and a selected music or sound.

The results in trials conducted so far have shown this method and apparatus to be very effective indeed and able to provide an improved experience for beneficial effect to a human.

## Claims

1. A method of stimulation of the senses for a participant, the method including the steps of subjecting the participant to both visual and olfactory stimulation, the visual stimulation being provided by a source or sources of light of selected patterns and/or colour or colours being directed into an atmosphere of fine water droplets, there being at least one essential oil as an olfactory stimulant carried by the droplets, **characterised in that** the participant is supported either directly or indirectly by a body of liquid so as to float, and while floating, from time to time, the fine water droplets together with the essential oil of a first set is substantially extracted and replaced with a fresh set of fine water droplets and a further essential oil.

2. The method of stimulation of the senses for a participant as in claim 1, the method including the steps of the participant entering into and being substantially supported by the body of liquid, the body of liquid having a high level of a dissolved salt so that the participant may float in the liquid, and while floating, being subjected to both the visual and olfactory stimulation.

3. The method of stimulation of the senses for a participant as in either one of claims 1 or 2, further **characterised in that** a diffuse colour effect is achieved by introducing the fine water droplets into an area immediately above the participant where the coloured lighting and sound are introduced as matching pairs.

4. The method of stimulation of the senses for a participant, as in any of the preceding claims, wherein the method includes a programmed sequence, wherein fine water droplets carrying a first selected essential oil are bathed with a selected colour of light and during which time there is also an appropriate audio stimulation of a selected music or sound selected for this purpose.

5. The method as in claim 4, further **characterised in that** the fine water droplets together with the essential oil being either with that fine water droplets or injected in cooperation with the fine water droplets is extracted and a replacement fine water droplets insertion is provided which also then carries a further selected essential oil and this is then triggered together with appropriate matching further selected colour bathing of the fine water droplets and an audio signal either music or selected noise.

6. The method as in claim 5, further **characterised in that** this combination of steps is further repeated for a selected number of times with, in each case, a different essential oil.

7. The method of stimulation of the senses for a participant, as in any one of the preceding claims, wherein the body of liquid and an area above the same into which the fine water droplets and the essential oil or oils are to be introduced are both within an enclosure.

8. The method as in claim 7, further **characterised in that** there is included the further step that during at least some of the period during which a first of the fine droplets have been introduced and are left to remain within the enclosure, there is also provided an input of an audible sound.

9. The method as in claim 8, further **characterised in that** there is included the further step that the audible sound is in the form of music which has been selected to be appropriate to accompany the essential oil or combination of oils at the time.

10. The method as in any one of claims 7 to 9, further **characterised in that** there is included the further step that there is a source of light which is coloured so that the coloured light is directed to shine within the enclosure at least while the fine water droplets are suspended in the atmosphere within the enclosure, and the colour or colours of such light are selected to be appropriate to accompany the essential oil or combination of oils within the enclosure at the time.

11. The method as in any one of the preceding claims, further comprised in that there is included the further step that the pattern of introducing and extracting an atmosphere of fine water droplets and an accompanying essential oil or oils combination is repeated a plurality of times with a period between such different sets of atmosphere to allow for effective extraction and then further introduction of fresh fine water droplets.

12. An apparatus for effecting a beneficial sensory input for a participant which includes a means to effect an input of fine water droplets into an area immediately above the body of liquid, means adapted to extract such fine water droplets, means to effect, in aerosol manner, input of each of a selected essential oil into the area above the body of liquid, means to effect each of a selected lighting either colour and/or pattern of the area above the body of liquid, and means to effect a sound input into the area for the participant to hear, **characterised in that** the apparatus includes a body of liquid within which the participant can float and means to effect in synchronised manner the input, extraction and replacement of fine water droplets in conjunction with the essential oils, noise and colours.

13. An apparatus as in claim 12, further **characterized in that** the body of liquid is a floatation pool located within an enclosure.

14. The apparatus as in claim 13, further **characterised in that** there is provided means to effect a sequenced arrangement for introduction and extraction of fine water droplets together with a sequence of introduction of a selected essential oil or oils combination with each further introduction of fine water droplets.

15. The apparatus as in any one of claims 13 or 14, further **characterised in that** there are means to introduce the essential oil or oils into the stream of fine water particles as they are introduced into the enclosure so that at least during some of the time of such introduction, the essential oil or oils combination will be intimately mixed with such fine water particles or droplets.

16. The apparatus as in any one of claims 14 or 15, further **characterised in that** there are means to effect a source of light which will effect a lighting of the atmosphere within the enclosure and there are means to effect a colouring of such light which is selected for each of the introduced essential oils.

17. The apparatus as in any one of claims 14 or 15, further **characterised in that** there are means to effect a heating of the liquid within the body of water and means to maintain this at a selected temperature which is approximately body temperature of a human body.

18. The apparatus as in any one of claims 14 or 15, further **characterised in that** the body of liquid has a high salt content and such salt is Epsom salts which is to an extent that a human body floating will have a substantive proportion of the body above an upper level of the liquid.

19. The apparatus as in any one of claims 14 or 15, further **characterised in that** there is a sequence controller which is adapted to effect during a selected time introduction of fine water droplets with an essential oil or oils combination, then a period during which the fine water droplets be allowed then to remain dormant within the enclosure while the colours are shone through the atmosphere of the enclosure and the sound is played, and there is then a period for extraction and reinsertion of a fresh set of fine water droplets together with a different set of essential oil or oils combination and at the same time a change in the audible sounds and also the colours and patterns selected for this particular further essential oil.

## Patentansprüche

1. Verfahren zur Stimulierung der Sinne eines Teilnehmers, wobei der Teilnehmer sowohl einer visuellen als auch einer olfaktorischen Stimulierung unterzogen wird, wobei die visuelle Stimulierung durch eine Quelle oder Quellen von Licht mit gewählten Mustern und/oder gewählter Farbe oder Farben bereitgestellt wird, das in eine Atmosphäre aus feinen Wassertröpfchen gerichtet wird, wobei mindestens ein ätherisches Öl als eine olfaktorische Stimulanz von den Tröpfchen getragen wird, **dadurch gekennzeichnet, dass** der Teilnehmer direkt oder indirekt von einer Flüssigkeitsmasse gestützt wird, so dass er von ihr getragen wird, wobei während dieses Getragenwerdens von Zeit zu Zeit die feinen Wassertröpfchen zusammen mit dem ätherischen Öl eines ersten Satzes im Wesentlichen abgezogen und durch einen frischen Satz feiner Wassertröpfchen und eines weiteren ätherischen Öls ersetzt werden.

2. Verfahren zur Stimulierung der Sinne eines Teilnehmers nach Anspruch 1, wobei sich der Teilnehmer in die Flüssigkeitsmasse begibt und im Wesentlichen durch sie gestützt wird, wobei der Gehalt der Flüssigkeitsmasse an gelöstem Salz hoch ist, so dass die Flüssigkeit den Teilnehmer tragen kann, wobei der Teilnehmer während des Getragenwerdens sowohl visueller als auch olfaktorischer Stimulierung unterzogen wird.

3. Verfahren zur Stimulierung der Sinne eines Teilnehmers nach Anspruch 1 oder 2, ferner **dadurch gekennzeichnet, dass** eine diffuse Farbwirkung erzielt wird, indem die feinen Wassertröpfchen in einen unmittelbar über dem Teilnehmer liegenden Bereich eingeführt werden, in dem das farbige Licht und Klang als zusammenpassende Paare eingeführt werden.

4. Verfahren zur Stimulierung der Sinne eines Teilnehmers nach einem der vorhergehenden Ansprüche, wobei das Verfahren eine programmierte Sequenz umfasst, bei der die ein erstes gewähltes ätherisches Öl tragenden feinen Wassertröpfchen in eine gewählte Lichtfarbe getaucht werden, wobei währenddessen auch eine angemessene Audiostimulierung durch gewählte Musik oder für diesen Zweck gewählte Klänge stattfindet.

5. Verfahren nach Anspruch 4, ferner **dadurch gekennzeichnet, dass** die feinen Wassertröpfchen zusammen mit dem ätherischen Öl, das entweder mit den feinen Wassertröpfchen zusammen ist oder in Zusammenwirkung mit den feinen Wassertröpfchen eingespritzt wird, abgezogen werden und dass feine Ersatzwassertröpfchen eingeführt werden, die ebenfalls ein weiteres gewähltes ätherisches Öl tragen, und dies wird dann zusammen mit angemessenem passendem weiterem Tauchen der feinen Wassertröpfchen in farbiges Licht und einem Tonsignal, das entweder aus Musik oder einem gewählten Geräusch besteht, ausgelöst.

6. Verfahren nach Anspruch 5, ferner **dadurch gekennzeichnet, dass** diese Kombination aus Schritten ferner gewählt oft mit jeweils einem anderen ätherischen Öl wiederholt wird.

7. Verfahren zur Stimulierung der Sinne eines Teilnehmers nach einem der vorhergehenden Ansprüche, wobei sich sowohl die Flüssigkeitsmasse als auch ein darüberliegender Bereich, in den die feinen Wassertröpfchen und das bzw. die ätherische(n) Öl(e) einzuführen sind, in einer Einfassung befinden.

8. Verfahren nach Anspruch 7, ferner **dadurch gekennzeichnet, dass** es einen weiteren Schritt enthält, bei dem während mindestens eines Teils des Zeitraums, während dessen erste der feinen Tröpfchen in die Einfassung eingeführt und dort belassen wurden, auch für eine Eingabe von einem hörbaren Klang gesorgt wird.

9. Verfahren nach Anspruch 8, ferner **dadurch gekennzeichnet, dass** es einen weiteren Schritt enthält, bei dem der hörbare Klang in der Form von gewählter Musik ist, die zum Zusammenspiel mit dem ätherischen Öl oder einer Kombination aus Ölen jeweils angemessen ist.

10. Verfahren nach einem der Ansprüche 7 bis 9, ferner **dadurch gekennzeichnet, dass** es einen weiteren Schritt enthält, bei dem eine Quelle farbigen Lichts vorliegt, so dass man das farbige Licht in der Einfassung mindestens während der Suspendierung der feinen Wassertröpfchen in der Atmosphäre in der Einfassung scheinen lässt, und wobei die Farbe oder Farben dieses Lichts so gewählt wird bzw. werden, dass sie dem ätherischen Öl oder einer Kombination aus Ölen in der Einfassung jeweils angemessen ist bzw. sind.

11. Verfahren nach einem der vorhergehenden Ansprüche, ferner mit dem weiteren Schritt, bei dem das Muster aus Einführen und Abziehen einer Atmosphäre aus feinen Wassertröpfchen und einem begleitenden ätherischen Ö1 oder einer begleitenden Kombination aus ätherischen Ölen mehrere Male wiederholt wird, wobei zwischen diesen unterschiedlichen Sätzen von Atmosphären ein Zeitraum liegt, der das wirksame Abziehen und dann das weitere Einführen von frischen feinen Wassertröpfchen gestattet.

12. Vorrichtung zum Bewirken einer günstigen sensorischen Eingabe für einen Teilnehmer, die Folgendes umfasst: ein Mittel zum Bewirken einer Eingabe von feinen Wassertröpfchen in einen Bereich unmittelbar über der Flüssigkeitsmasse, Mittel, die dazu geeignet sind, diese feinen Wassertröpfchen abzuziehen, Mittel, um nach Art eines Aerosols die Eingabe von jeweils einem gewählten ätherischen Öl in den Bereich über der Flüssigkeitsmasse zu bewirken, Mittel zum Bewirken jeweils einer gewählten Beleuchtung in einer Farbe und/oder einem Muster des Bereichs über der Flüssigkeitsmasse und Mittel zum Bewirken einer für den Teilnehmer hörbaren Klangeingabe in den Bereich, **dadurch gekennzeichnet, dass** die Vorrichtung eine Flüssigkeitsmasse aufweist, die den Teilnehmer tragen kann, sowie Mittel, um auf synchronisierte Weise die Eingabe, das Abziehen und das Ersetzen feiner Wassertröpfchen in Verbindung mit den ätherischen Ölen, Geräuschen und Farben zu bewirken.

13. Vorrichtung nach Anspruch 12, ferner **dadurch gekennzeichnet, dass** es sich bei der Flüssigkeitsmasse um einen in der Einfassung angeordneten Flotationstank handelt.

14. Vorrichtung nach Anspruch 13, ferner **dadurch gekennzeichnet, dass** Mittel vorgesehen sind, um eine Folgeanordnung für das Einführen und Abziehen von feinen Wassertröpfchen zusammen mit einer Folge zur Einführung eines gewählten ätherischen Öls oder einer Kombination von gewählten ätherischen Ölen mit jeder weiteren Einführung von feinen Wassertröpfchen zu bewirken.

15. Vorrichtung nach Anspruch 13 oder 14, ferner **dadurch gekennzeichnet, dass** Mittel zum Einführen des ätherischen Öls oder der ätherischen Öle in den Strom feiner Wasserpartikel, während diese in die Einfassung eingeführt werden, vorliegen, so dass das ätherische Öl oder die Kombination aus ätherischen Ölen mindestens während einer gewissen Zeit dieser Einführung innig mit diesen feinen Wasserpartikeln oder -tröpfchen vermischt wird.

16. Vorrichtung nach Anspruch 14 oder 15, ferner **dadurch gekennzeichnet, dass** Mittel zur Bereitstellung einer Lichtquelle, die eine Beleuchtung der Atmosphäre in der Einfassung bewirkt, sowie Mittel zum Bewirken einer Färbung dieses Lichts vorliegen, wobei die Farbe jeweils für die eingeführten ätherischen Öle gewählt wird.

17. Vorrichtung nach Anspruch 14 oder 15, ferner **dadurch gekennzeichnet, dass** Mittel zum Bewirken eines Heizens der Flüssigkeitsmasse sowie Mittel vorliegen, um sie auf einer gewählten Temperatur zu halten, bei der es sich um ungefähr die menschliche Körpertemperatur handelt.

18. Vorrichtung nach Anspruch 14 oder 15, ferner **dadurch gekennzeichnet, dass** die Flüssigkeitsmasse einen hohen Salzgehalt hat und dass es sich bei dem Salz um Bittersalz handelt, wobei der Salzgehalt so hoch ist, dass sich ein wesentlicher Teil eines darin schwimmenden menschlichen Körpers über dem oberen Pegel der Flüssigkeit befindet.

19. Vorrichtung nach Anspruch 14 oder 15, ferner **dadurch gekennzeichnet, dass** eine Folgesteuerung vorliegt, die geeignet ist, während einer gewählten Zeit die Einführung feiner Wassertröpfchen mit einem ätherischen Öl oder einer Kombination aus ätherischen Ölen, danach einen Zeitraum, während dessen die feinen Wassertröpfchen dann in der Einfassung ruhen könncn, während die Farben durch die Atmosphäre der Einfassung gestrahlt werden und der Klang gespielt wird, und dann einen Zeitraum zum Abziehen und erneuten Einführen eines frischen Satzes feiner Wassertröpfchen zusammen mit einem anderen Satz eines ätherischen Öls oder einer Kombination aus ätherischen Ölen zu bewirken, wobei sich gleichzeitig die hörbaren Klänge sowie die für dieses bestimmte weitere ätherische Öl gewählten Farben und Muster ändern.

## Revendications

1. Procédé de stimulation des sens d'un participant, le procédé comportant les étapes consistant à soumettre le participant à une stimulation à la fois visuelle et olfactive, la stimulation visuelle étant fournie par une ou plusieurs sources de lumière présentant des motifs et/ou une ou plusieurs couleurs sélectionné(e)s, dirigée dans une atmosphère de fines gouttelettes d'eau, en présence d'au moins une huile essentielle en tant que stimulant olfactif porté par les gouttelettes, **caractérisé en ce que** le participant est soutenu directement ou indirectement par une masse de liquide de façon à flotter et que de temps à autre, tandis qu'il flotte, les fines gouttelettes d'eau ainsi que l'huile essentielle d'un premier ensemble sont sensiblement extraites et remplacées par un nouvel ensemble de fines gouttelettes d'eau et une autre huile essentielle.

2. Procédé de stimulation des sens d'un participant selon la revendication 1, le procédé comportant les étapes consistant pour le participant à entrer dans la masse de liquide et à être sensiblement soutenu par celle-ci, la masse de liquide comportant un niveau élevé d'un sel dissous de telle sorte que le participant puisse flotter dans le liquide, et à être soumis, tandis qu'il flotte, à la stimulation à la fois visuelle et olfactive.

3. Procédé de stimulation des sens d'un participant selon l'une quelconque des revendications 1 et 2, **caractérisé de plus en ce qu'**un effet de couleur diffuse est obtenu en introduisant les fines gouttelettes d'eau dans une région située immédiatement au-dessus du participant où l'éclairage coloré et le son sont introduits par paires correspondantes.

4. Procédé de stimulation des sens d'un participant selon l'une quelconque des revendications précédentes, le procédé comprenant une séquence programmée, les fines gouttelettes d'eau porteuses d'une première huile essentielle sélectionnée étant baignées dans une couleur de lumière sélectionnée et une stimulation auditive appropriée étant assurée pendant cette période par une musique ou un son sélectionné(e) à cet effet.

5. Procédé selon la revendication 4, **caractérisé de plus en ce que** les fines gouttelettes d'eau combinées à l'huile essentielle qui est soit ajoutée aux fines gouttelettes d'eau, soit injectée en coopération avec les fines gouttelettes d'eau, sont extraites et qu'on assure une insertion de fines gouttelettes d'eau de remplacement, qui porte alors elle-même une autre huile essentielle sélectionnée, celle-ci étant alors déclenchée conjointement à un autre bain de couleur sélectionné approprié correspondant des fines gouttelettes d'eau et à un signal audio constitué de musique ou d'un bruit sélectionné.

6. Procédé selon la revendication 5, **caractérisé de plus en ce que** cette combinaison d'étapes est en outre répétée un nombre choisi de fois avec, dans chaque cas, une huile essentielle différente.

7. Procédé de stimulation des sens d'un participant selon l'une quelconque des revendications précédentes, la masse de liquide et une région située au-dessus de celle-ci dans laquelle les fines gouttelettes d'eau et l'huile ou les huiles essentielles doivent être introduites se trouvant toutes deux à l'intérieur d'une enceinte.

8. Procédé selon la revendication 7, **caractérisé de plus en ce qu'**il comprend l'étape supplémentaire consistant, durant au moins une partie de la période pendant laquelle on a introduit les premières des fines gouttelettes et on les laisse séjourner à l'intérieur de l'enceinte, à assurer également une entrée de son audible.

9. Procédé selon la revendication 8, **caractérisé de plus en ce qu'**il comprend l'étape supplémentaire lors de laquelle le son audible se présente sous la forme d'une musique qui a été sélectionnée de façon à être appropriée pour accompagner l'huile ou la combinaison d'huiles essentielles à l'instant en question.

10. Procédé selon l'une quelconque des revendications 7 à 9, **caractérisé de plus en ce qu'**il comprend l'étape supplémentaire faisant intervenir une source de lumière colorée de telle sorte que la lumière colorée est dirigée de façon à briller à l'intérieur de l'enceinte au moins pendant que les fines gouttelettes d'eau sont suspendues dans l'atmosphère à l'intérieur de l'enceinte, et la ou les couleurs de cette lumière sont sélectionnées de façon à être appropriées pour accompagner l'huile ou la combinaison d'huiles essentielles se trouvant à l'intérieur de l'enceinte à l'instant en question.

11. Procédé selon l'une quelconque des revendications précédentes, **caractérisé de plus en ce qu'**il comprend l'étape supplémentaire lors de laquelle le schéma d'introduction et d'extraction d'une atmosphère de fines gouttelettes d'eau et d'une huile ou d'une combinaison d'huiles essentielles l'accompagnant est répété une pluralité de fois avec une période entre ces différents ensembles d'atmosphère afin de permettre une extraction, puis une introduction supplémentaire efficaces de nouvelles fines gouttelettes d'eau.

12. Appareil destiné à effectuer une sollicitation sensorielle bénéfique pour un participant, comprenant un moyen destiné à effectuer une introduction de fines gouttelettes d'eau dans une région située immédiatement au-dessus de la masse de liquide, des moyens prévus pour extraire lesdites fines gouttelettes d'eau, des moyens destinés à effectuer, de manière à créer un aérosol, l'introduction de chaque huile essentielle sélectionnée dans la région située au-dessus de la masse de liquide, des moyens destinés à réaliser chaque couleur et/ou motif d'éclairage sélectionné de la région située au-dessus de la masse de liquide, et des moyens destinés à effectuer une introduction de son dans la région afin que le participant l'entende, **caractérisé en ce que** l'appareil comprend une masse de liquide à l'intérieur de laquelle le participant peut flotter et des moyens d'effectuer de manière synchronisée l'introduction, l'extraction et le remplacement de fines gouttelettes d'eau en conjonction avec les huiles essentielles, le bruit et les couleurs.

13. Appareil selon la revendication 12, **caractérisé de plus en ce que** la masse de liquide est un bassin de flottaison situé à l'intérieur d'une enceinte.

14. Appareil selon la revendication 13, **caractérisé de plus par** la présence de moyens destinés à effectuer un agencement séquentiel pour l'introduction et l'extraction de fines gouttelettes d'eau, conjointement à une séquence d'introduction d'une huile ou d'une combinaison d'huiles essentielles sélectionnées avec chaque nouvelle introduction de fines gouttelettes d'eau.

15. Appareil selon l'une quelconque des revendications 13 et 14, **caractérisé de plus par** la présence de moyens destinés à introduire l'huile ou les huiles essentielles dans le flux de fines particules d'eau tandis qu'elles sont introduites dans l'enceinte, de telle sorte que pendant au moins une partie de la durée de ladite introduction, l'huile ou la combinaison d'huiles essentielles soient intimement mélangées auxdites fines particules ou gouttelettes d'eau.

16. Appareil selon l'une quelconque des revendications 14 et 15, **caractérisé de plus par** la présence de moyens destinés à créer une source de lumière qui effectuera un éclairage de l'atmosphère à l'intérieur de l'enceinte et par la présence de moyens destinés à effectuer une coloration de ladite lumière, sélectionnée pour chacune des huiles essentielles introduites.

17. Appareil selon l'une quelconque des revendications 14 et 15, **caractérisé de plus par** la présence de moyens destinés à effectuer un chauffage du liquide au sein de la masse de liquide et de moyens destinés à maintenir celle-ci à une température sélectionnée qui est approximativement la température corporelle d'un corps humain.

18. Appareil selon l'une quelconque des revendications 14 et 15, **caractérisé de plus en ce que** la masse de liquide présente une forte teneur en sel et que ledit sel est constitué de sels d'Epsom, au point qu'un corps humain flottant présentera une proportion substantielle du corps au-dessus d'un niveau supérieur du liquide.

19. Appareil selon l'une quelconque des revendications 14 et 15, **caractérisé de plus par** la présence d'un contrôleur de séquences prévu pour effectuer, pendant un temps sélectionné, l'introduction des fines gouttelettes d'eau avec une huile ou une combinaison d'huiles essentielles, puis une période pendant laquelle on laisse séjourner les fines gouttelettes d'eau de façon latente à l'intérieur de l'enceinte tandis que les couleurs sont projetées à travers l'atmosphère de l'enceinte et que l'on produit le son, suivie d'une période d'extraction et de réinsertion d'un nouvel ensemble de fines gouttelettes d'eau associées à un ensemble différent d'huile ou de combinaison d'huiles essentielles, au même instant qu'un changement des sons audibles ainsi que des couleurs et des motifs sélectionnés pour ladite autre huile essentielle en question.
